# EUROPÄISCHE PATENTANMELDUNG

(11) **EP 2 514 391 A1**
(43) Veröffentlichungstag der Anmeldung: **24.10.2012**
(21) Anmeldenummer: 11163204.8
(22) Anmeldetag: 20.04.2011
(51) Int. Cl.: A61F 2/38

(54) **Gelenkprothese mit einem Beugescharnier mit Spreizachse**

(71) Anmelder: Deru GmbH, 22844 Norderstedt (DE)
(72) Erfinder: Bartels, Carolin, 25355 Barmstedt (DE); Dmuschewsky, Klaus, 22397 Hamburg (DE); Iredi, Marco, 22848 Norderstedt (DE)
(74) Vertreter: Glawe, Delfs, Moll

(57) **Zusammenfassung**

Die Erfindung betrifft eine Gelenkprothese mit einem Beugescharnier, das von einer Scharniergabel und einem Achsbolzen (4) gebildet ist, der zwei Achsstümpfe (42, 43) umfasst, die zum Einsetzen in einer in einem Koppelstück (3) eingezogenen Montagestellung und nach Implantation durch Bewegung in Achsrichtung in fluchtende Scharnierbohrungen der Scharniergabel in einer expandierten Stellung angeordnet sind, wobei der Achsbolzen (4) zwei Lagerbereiche (41) an seinen Enden und einen dazwischen liegenden Fügebereich (40) aufweist, und er im Fügebereich (41) längs einer in Achsrichtung verlaufenden Ebene (49) getrennt ist, welche den Mantel des Achsbolzens (4) an zwei Stellen schneidet.

## Beschreibung

Die Erfindung betrifft eine Gelenkendoprothese mit einem Beugescharnier, dessen Achsbolzen zwei Achsstümpfe umfasst, die zum Einsetzen in einer in das Koppelstück eingezogenen Montagestellung und nach Einsetzen des Mittelteils durch Bewegung in ihrer Längsrichtung in fluchtende Scharnierbohrungen der Scharniergabel in einer gespreizten Stellung angeordnet sind.

Derartige Endoprothesen werden insbesondere als Prothesen für Kniegelenke verwendet. Diese sind wegen ihrer hohen Belastung durch das Körpergewicht des Patienten und wegen ihres komplexen Bewegungsablaufs vergleichsweise anfällig gegenüber Störungen durch Verschleiß oder Krankheit. Der Ersatz eines Kniegelenks durch eine Endoprothese ist eine aufwendige und für den Patienten belastende Operation. Es ist daher erwünscht, dass die Endoprothese möglichst einfach und störunanfällig zu implantieren ist, um Komplikationen zu vermeiden.

Dazu ist eine Gelenkendoprothese der eingangs genannten Art bekannt geworden, die einen spreizbaren Achsbolzen aufweist (EP 1 381 335 B1). Sie umfasst eine tibiale Komponente und eine femorale Komponente, die über ein Koppelstück gelenkig miteinander verbunden sind. Es ist ein Beugegelenk und ein Rotationsgelenk gebildet. Das Beugegelenk erlaubt eine Flexion und Extension des Knies. Dazu weist das Koppelstück ein Achsauge auf, in das ein Achsbolzen eingesetzt ist, der aus Achsstümpfen besteht. Die Achsstümpfe sind mit einander komplementären koaxialen Ausnehmungen versehen, so dass sie längs ihrer gemeinsamen Mittelachse teleskopartig aufeinander zu oder voneinander weg bewegt werden können. Zum Einsetzen sind die Achsstümpfe ganz aufeinander zu bewegt und befinden sich so in einer Montageposition, bei der die Achsstümpfe in das Koppelstück eingezogen sind. Nach dem Montieren des Koppelstücks in dem Gegenstück, einer Scharniergabel an der femoralen Komponente, werden sie gespreizt und expandieren so in die miteinander fluchtenden Bohrungen in der Scharniergabel. Damit ist das Koppelstück schwenkbeweglich mit der femoralen Komponente verbunden. Dieser Achsbolzen ermöglicht eine leichtere Montage, allerdings kann es im Fall einer Verklemmung der Achsstümpfe zu Komplikationen kommen.

Der Erfindung liegt die Aufgabe zugrunde, ausgehend von dem zuletzt genannten Stand der Technik eine verbesserte Gelenkendoprothese zu schaffen, die diesen Nachteil vermeidet.

Die erfindungsgemäße Lösung liegt in den Merkmalen des unabhängigen Anspruchs. Vorteilhafte Weiterbildungen sind Gegenstand der abhängigen Ansprüche.

Bei einer Gelenkprothese mit einem Beugescharnier, das von einer Scharniergabel und einem Achsbolzen gebildet ist, der zwei Achsstümpfe umfasst, die zum Einsetzen in einer in einem Koppelstück eingezogenen Montagestellung und nach Implantation durch Bewegung in Achsrichtung in fluchtende Scharnierbohrungen der Scharniergabel in einer expandierten Stellung angeordnet sind, ist erfindungsgemäß vorgesehen, dass der Achsbolzen zwei Lagerbereiche an seinen Enden und einen dazwischen liegenden Fügebereich aufweist, und er im Fügebereich längs einer in Achsrichtung verlaufenden Ebene getrennt ist, welche den Mantel des Achsbolzens an zwei Stellen schneidet.

Die Erfindung beruht auf dem Gedanken, den Fügebereich der beiden Achsstümpfe in zwei Halbschäfte zu teilen, und zwar mittels einer Ebene, die längs der Mittelachse läuft und den Mantel des Achsbolzens an zwei Stellen schneidet. In der Regel ist dies die Mittelebene, zwingend ist dies jedoch nicht. Sie kann aus mehreren Teilebenen zusammengesetzt sein, jedoch wird sie in den meisten Fällen durchgängig sein. Da sie von einer auf die andere Seite läuft, ist sie maximal breit, nämlich so groß wie der Durchmesser der Achse im Fügebereich. Damit ergibt sich eine über den ganzen Durchmesser reichende Ebene, längs der die beiden Achsstümpfe mit maximaler Breite geführt sind. Die breite Führung sorgt für günstige Reibverhältnisse und vermeidet so ein Verklemmen. Da überdies die Führungsebene den Mantel an mindestens zwei Stellen schneidet, besteht ein Freiheitsgrad zum Ausgleich von Verkantungen. Dies stellt einen erheblichen Vorteil zum Stand der Technik mit geschlossenen - etwa hohlzylinderartigen - Führungsebenen dar, die aufgrund von Verkantungen zum Blockieren neigen können. Es ergeben sich mit der Erfindung somit erhebliche Vorteile sowohl bei der Erstimplantation wie auch bei eventuell nachfolgend erfolgenden Revisionen.

Vorzugsweise sind die Achsstümpfe in ihrem Fügebereich komplementär zueinander. Damit kann durch einfaches Zusammenstecken dieselbe Außenkontur erreicht werden, wie sie die herkömmlicherweise verwendeten ungeteilten Achsbolzen aufweist. Der erfindungsgemäße Achsbolzen kann so in herkömmlichen Koppelstücken verwendet sein. Besonders bevorzugt ist es, wenn die Achsstümpfe nicht nur komplementär, sondern sogar formgleich sind. Bei einer bewährten Ausführungsform weisen die Achsstümpfe Halbzapfen im Fügebereich auf. Beispielsweise ist der Querschnitt im Fügebereich halbmondartig.

Das Koppelstück weist mit Vorteil ein Trennelement mit einer Durchbrechung zur Aufnahme des Fügebereichs der Achsstümpfe auf, wobei die Durchbrechung unrund ist. Damit kann eine Verdrehsicherung der beiden Achsstümpfe in dem Koppelstück erreicht werden. Eine gesonderte Verdrehsicherung, beispielsweise in Gestalt einer leicht verlierbaren Schraube, ist damit nicht mehr erforderlich.

An dem Koppelstück kann eine Verschiebesicherung angeordnet sein. Sie wirkt auf die beiden Achsstümpfe und sichert sie in ihrer gespreizten Position. Damit ist eine Feder, wie sie im Stand der Technik zur Spreizung vorgesehen ist, nicht erforderlich. Im Fall einer Revision können die Achsstümpfe so durch einfaches Entfernen der Verschiebesicherung in die ungespreizte Montagestellung verschoben werden, ohne dass dazu die gegenwirkende Kraft der Spreizfeder dauerhaft überwunden zu werden braucht.

Um auf die Achsstümpfe in dem Koppelstück einwirken zu können, sind an jenem vorzugsweise für jeden Achsstumpf ein Zugangsschlitz vorgesehen. Er ist so orientiert, dass seine längliche Erstreckung in Achsrichtung liegt und eine solche Erstreckung aufweist, die mindestens so groß ist wie die Strecke, über die die Achsstümpfe zu spreizen sind. Es ist ein Spreizinstrument vorgesehen, das in die Zugangsschlitze einsteckbar ist zum Verschieben der Achsstümpfe. Zweckmäßigerweise ist es bügelartig ausgeformt mit zwei freien Enden zum Einstecken in die Zugangsschlitze, wobei die freien Enden vorzugsweise in einem Winkel von 30 Grad bis 75 Grad zueinander stehen können. Es kann aus einem Drahtstück gebildet sein, in dessen hinteren Bereich der Draht eine von den freien Enden weg weisende Dreipunktschlaufe bildet. Auf sie kann eine Schiebehülse aufgesteckt sein, die aus einer Ruhestellung, bei der die freien Enden nahe beieinander stehen, die in eine bewegliche Spreizposition gelangen, bei der die freien Enden relativ voneinander wegbewegt sind.

Am Spreizinstrument kann eine Befestigungsführung angeordnet sein. Sie ist als eine Hülse ausgeführt, in der eine Sperrschraube angeordnet ist. Zweckmäßigerweise weist sie dazu ein Innengewinde auf, in das die Sperrschraube eingedreht ist. Die Befestigungsführung ist so ausgerichtet, dass sie bei an das Koppelstück angesetztem Spreizinstrument auf die Achse zielt. Vorzugsweise ist die Länge der Sperrschraube größer, als es dem Abstand zwischen dem koppelstückseitigen Ende der Befestigungsführung und dem Fügebereich der Achsstümpfe entspricht. Damit ist sichergestellt, dass das Spreizinstrument mitsamt der Befestigungsführung erst abgenommen werden kann, wenn die Achsstümpfe in ihrer gespreizten Position gesichert sind.

Die Schiebehülse kann einen umlaufenden radialen Kragen aufweisen, der an seiner zu der Befestigungsführung weisenden Seite eine Ausnehmung aufweist. Damit ist ein Verschieben auch auf einem gekrümmten Bügel des Spreizinstruments sowie ein gerader Zugangsweg für einen Schraubendreher zu der in der Befestigungsführung angeordneten Sperrschraube gewährleistet.

Weiter kann eine Revisionszange vorgesehen sein. Sie weist an ihrem hinteren Ende Handgriffe und an ihrem vorderen Ende Aufnahmefinger auf, die zum Durchstecken durch die Zugangsschlitze ausgebildet und im Querschnitt oval sind. Die Aufnahmefinger divergieren nach vorne mit einem Winkel von vorzugsweise zwischen 5 und 25 Grad.

Die Erfindung wird nachfolgend unter Bezugnahme auf die beigefügte Zeichnung näher erläutert, in der ein vorteilhaftes Ausführungsbeispiel dargestellt ist. Es zeigen:

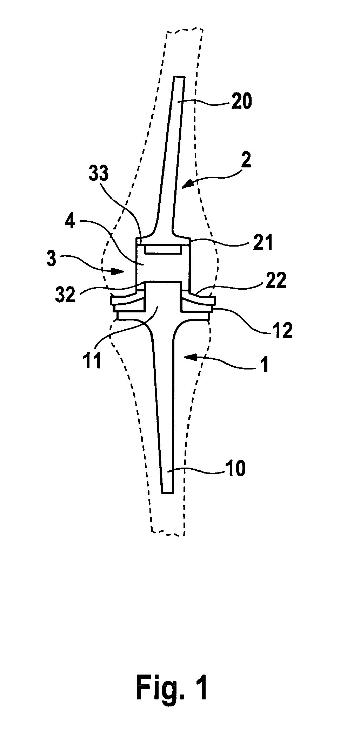

- Fig. 1: eine Rückansicht einer Gelenkprothese;
- Fig. 2: eine perspektivische Ansicht des Koppelstücks;
- Fig. 3a-c: Schnittansichten des Koppelstücks;
- Fig. 4a-c: Detailansichten eines Achsstumpfs;
- Fig. 5: eine Aufsicht auf ein Spreizinstrument;
- Fig. 6: eine Seitenansicht im Schnitt von dem Kippel-stück mit Spreizinstrument;
- Fig. 7: eine perspektivische Ansicht zu Fig. 6; und
- Fig. 8a,: b eine Revisionszange in einer perspektivischen Ansicht und einer Detailansicht.

Die Endoprothese gemäß einem Ausführungsbeispiel der Erfindung wird anhand einer Kniegelenkendoprothese erläutert. Die Endoprothese eines Kniegelenks besteht im Wesentlichen aus zwei Komponenten 1, 2, von denen eine als tibiale Komponente 1 und die andere als femorale Komponente 2 ausgebildet ist. Bei der femoralen Komponente 2 schließt sich an einen Stiel 20, der in einen Oberschenkelknochen eines Patienten eingesetzt wird, eine femorale Lagerhälfte 21 an, welche über zwei gabelartig zur tibialen Komponente 1 vorspringende kondylenartige Gleitkufen 22 verfügt. Diese stützen sich auf einem Tibiaplateau 12 ab, das auf einer tibialen Lagerhälfte 11 angeordnet ist, welche über einen Stiel 10 an einem Unterschenkelknochen des Patienten befestigt ist.

Dazwischen ist ein Koppelstück 3 angeordnet, welches ein T-Stück 30 als Hauptkörper mit einem in seinem oberen Bereich angeordneten Aufnahmeauge 33 für einen Achsbolzen 4 und einem Lagerstift 31 zur Aufnahme in eine Lagerbuchse in der tibialen Lagerhälfte 11.

Ein erstes Lager (Flexionslager) ermöglicht eine Schwenkbewegung zwischen den Komponenten 1 und 2, realisiert also die Beugebewegung zwischen Ober- und Unterschenkel. Diese Schwenkbewegung um die Achse des Achsbolzens 4 bildet damit die erste Achse für die Bewegung der Kniegelenkprothese. Quer dazu ist der Stift 31 mit seiner Mittelachse ausgerichtet, welche eine zweite Achse bildet für die Rotationsbewegung, mit der sich der Femurteil 2 relativ zur Tibiakomponente 1 um die zweite Achse verdreht. Für dieses Rotationslager ragt der Stift 31 des Koppelstücks 3 in die Lagerbuchse hinein. Zwischen den beiden ist ein Lagereinsatz 32 angeordnet.

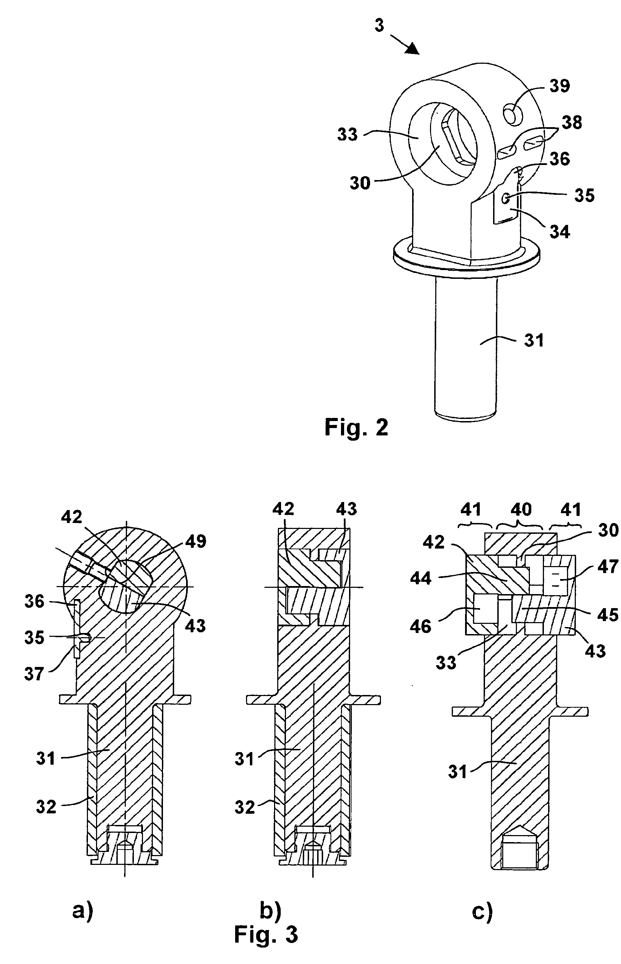

In dem Aufnahmeauge 33 ist der spreizbare Achsbolzen 4 angeordnet. Er weist mehrere Bereiche auf, je einen Lagerbereich 41 an beiden Enden und dazwischen einen Fügebereich 40. Er ist in Fig. 3b in seiner Montageposition dargestellt, in der er in das Aufnahmeauge 33 eingezogen ist, und in Fig. 3c in seiner Spreizposition, in der die Lagerbereiche 41 zu beiden Seiten aus dem Aufnahmeauge 33 herausragen. Er ist durch eine Durchbrechung eines Trennelements 30 in dem Aufnahmeauge 33 gesteckt, welche die Form eines abgeflachten Ovals aufweist und den Achsbolzen 4 an einer Verdrehung hindert.

An seiner Vorderseite (in der Darstellung in Figur 3a links) ist das Koppelstück 3 mit einer planen Aufnahmefläche 35 versehen. An ihrem oberen Ende ist eine Tasche 36 in dem Aufnahmeauge 33 ausgeformt, die an ihrem Grund plan in die Aufnahmefläche 34 übergeht. Im mittleren Bereich der Aufnahmefläche 34 ist eine Halteöffnung 35 vorgesehen, die als Sackloch ausgeführt ist. Eine Prallschutzplatte 37 kann auf die Aufnahmefläche 34 aufgesetzt und in die Tasche 36 eingeschoben sein. Dadurch ist die Tasche 36 mit ihrer oberen Kante gegenüber einem Abheben von der Aufnahmefläche 34 gesichert, und zwar auch unter Krafteinwirkung von vorne (in Figur 3a von links) beim Erreichen der gestreckten Anschlagsposition des Flexionslagers. Gegen ein Verschieben insbesondere nach unten ist die Prallschutzplatte 37 mittels eines an ihrer Rückseite ausgeformten Vorsprungs gesichert, der formschlüssig in die Halteöffnung 35 eingreift.

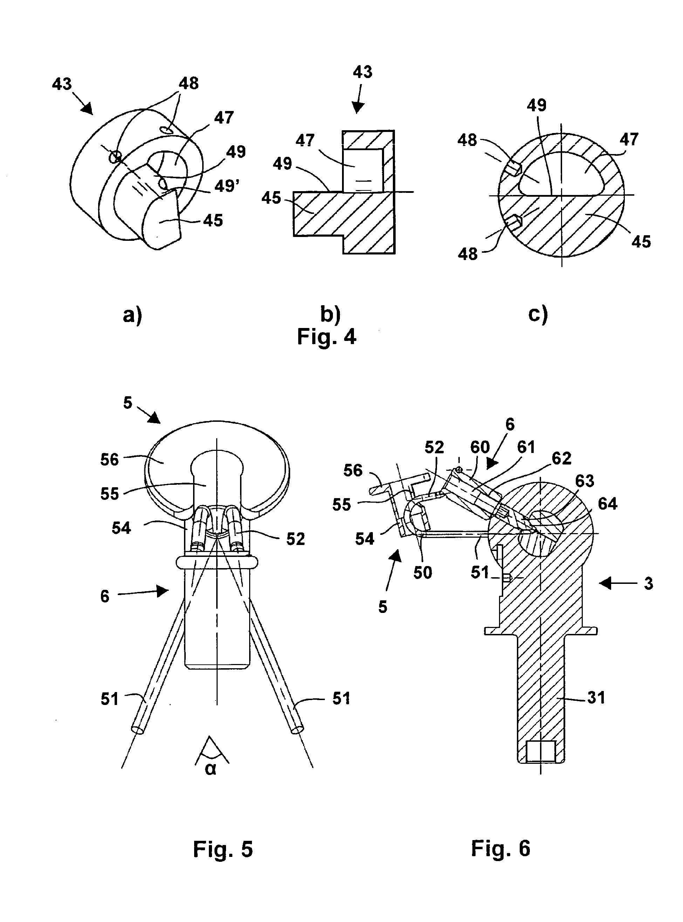

Der Achsbolzen 4 weist im Fügebereich 40 eine längs zu seiner Mittelachse verlaufende Trennebene 49 auf. Sie schneidet den Mantel im Fügebereich 40 an zwei diametral gegenüber liegenden Stellen (s. Fig. 3a). Der Achsbolzen 4 ist so geteilt in zwei Achsstümpfe 42, 43. Beide weisen einen vorspringenden Halbzapfen 44, 45 auf, die zusammengesetzt den Querschnitt des Achsbolzens 4 im Fügebereich 40 bilden. Weiter weist jeder der beiden Achsstümpfe 42, 43 eine halbmondförmige Vertiefung 46, 47 auf. Sie sind komplementär zu den Halbzapfen 44, 45 geformt, so dass jene bei zusammengeschobenen Achsstümpfen (für die Montageposition, wie in Fig. 3b dargestellt) in die Vertiefung 46, 47 eintauchen. In der gespreizten Position bewegen sich die Halbzapfen 44, 45 aus den Vertiefungen 46, 47 heraus, wobei sie durch die breite Trennebene 49 sicher geführt sind. Wegen dieser breiten Führung in einer planen Ebene kommt es bei dem Spreizen weder zu Verklemmungen noch zu Verkantungen; das gleiche gilt für ein Zusammenschieben im Fall einer Revision der Kniegelenkprothese. Der Aufbau der Achsstümpfe 42, 43 ist detailliert in Fig. 4 dargestellt. Sie weisen weiter an ihrem Mantel eine Aktuatoröffnung 48 auf, die zum Aufnehmen eines Spreizinstruments 5 ausgebildet ist. Vorzugsweise sind zwei Aktuatoröffnungen 48 je Achsstumpf vorgesehen, und zwar symmetrisch zur Trennebene 49. Verwendet wird jeweils eines, und zwar je nach Einbauposition. Bei dem dargestellten Ausführungsbeispiel sind die Achsstümpfe 42, 43 formgleich, d. h. das gleiche Element kann sowohl als Achsstumpf 42 wie auch als Achsstumpf 43 verwendet werden.

Um das Spreizen der Achsstümpfe 42, 43 zu bewirken, sind an dem Aufnahmeauge 33 zwei Zugangsschlitze 38 ausgebildet. Sie sind auf einer fluchtenden Linie mit einer Orientierung ihrer länglichen Erstreckung in Richtung der Mittelachse des Achsbolzens 4 ausgerichtet. Oberhalb von und mittig zwischen ihnen ist eine Befestigungsöffnung 39 vorgesehen. Durch die Zugangsschlitze 38 kann ein spitzes Instrument gesteckt werden, welches in eine der beiden Aktuatoröffnungen 48 eingreift. Durch Bewegen des Instruments nach außen wird der Achsstumpf 42 von dem anderen Achsstumpf entfernt, und umgekehrt.

Das dazu verwendete Spreizinstrument ist in Fig. 5 und 6 näher dargestellt. Es umfasst einen Drahtbügel 50 mit zwei freien Enden 51 an seinem vorderen Ende, die in einem Winkel α divergieren. Es bildet in seinem hinteren Bereich eine Dreipunktschlaufe, auf die eine Schiebehülse 54 aufgesteckt ist. Sie weist an ihrem den Enden 51 abgewandten Seite einen radial vorstehenden Kragen 56 auf, der mit einer Ausnehmung 55 versehen ist. Die Schiebehülse 54 ist zwischen ihrer Spreizposition, wie in Fig. 5 dargestellt, nach vorne verschieblich in eine Ruheposition, wodurch die freien Enden 51 in eine Position gedrückt sind, in der sie näher zusammenstehen. Hierzu ist eine gewisse Verstellkraft erforderlich, so dass die Schiebehülse 54 als Sicherung gegen ein unbeabsichtigtes Verschieben fungiert. Die Ausnehmung 55 stellt sicher, dass der Kragen 56 bei dem Verschieben nicht mit dem Drahtbügel 50 kollidiert. Außerdem sorgt sie für einen ungehinderten geraden Zugang zur Befestigungshülse 60.

Die Befestigungshülse 60 ist Teil einer Befestigungsführung 6, die am Ende des Drahtbügels 50 angeordnet ist. Die Befestigungshülse weist in ihrer inneren Öffnung 61 ein Innengewinde 62 auf, in das eine Sperrschraube 63 eingedreht ist. In der Ruheposition ragt die Sperrschraube 63 nur knapp mit ihrer Spitze 64 heraus, und zwar soweit, dass sie in die Öffnung 39 mit ihrem Gewinde fasst und die Befestigungsführung so an dem Koppelstück 3 sichert.

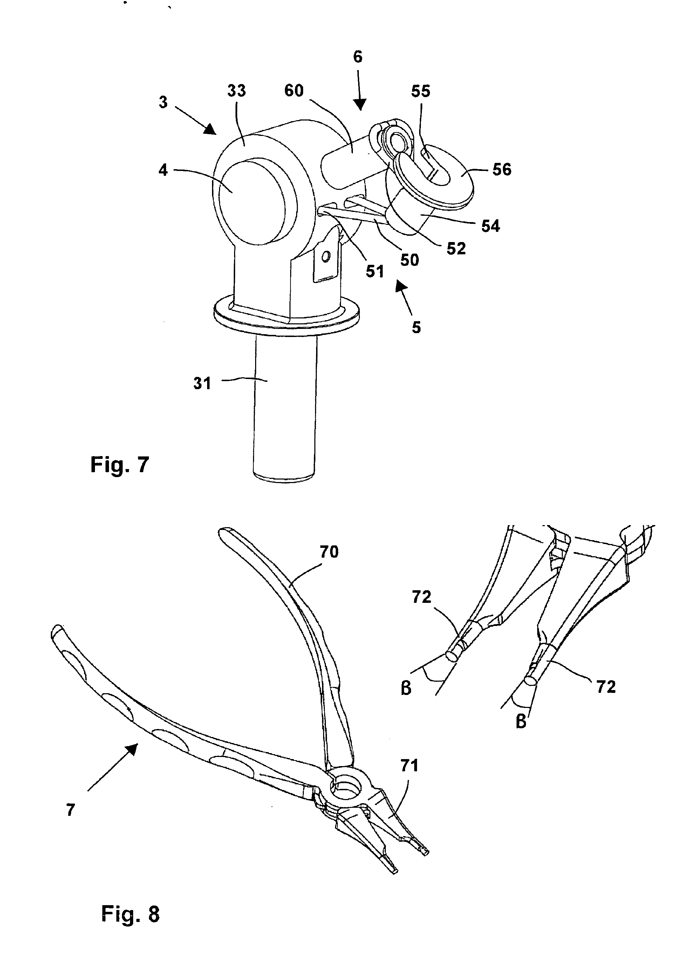

Bei der Montage wird zum Spreizen des Achsbolzens 4 die Schiebehülse 5 nach hinten bewegt, wodurch die durch die Zugangsschlitze 38 in die Aktuatoröffnungen 48 der Achsstümpfe 42, 43 gesteckten freien Enden 51 voneinander weg bewegt werden und so die Achsstümpfe 42, 43 auseinander spreizen. Haben sie ihre gespreizte Position erreicht (s. Fig. 3c und 7), so wird die Sperrschraube 63 weiter eingedreht, bis sie mit ihrer Spitze in der Trennebene 49 eintritt und die Achsstümpfe 42, 43 durch Klemmung und, bei Vorhandensein einer entsprechenden Aufnahmeöffnung 49', sogar formschlüssig sichert. Ist die Sperrschraube 63 ausreichend weit eingedreht, ist sie nicht mehr im Eingriff mit dem Innengewinde 62. Die Befestigungsführung 6 ist damit frei, und das Spreizinstrument 5 kann dann (erst) abgenommen werden. Damit ist sichergestellt, dass das Spreizinstrument erst dann abgenommen werden kann, wenn die Achsstümpfe 42, 43 gespreizt sind.

Bei einer Revision ist es erforderlich, die Achsstümpfe 42, 43 aus der gespreizten Position (s. Fig. 3c) wieder in die Montageposition (s. Fig. 3b) zurück zu bewegen. Dazu ist eine Revisionszange 7 vorgesehen mit einem Handgriff 70 im hinteren und Aufnahmefingern 70 an Backen 71 am vorderen Ende (s. Fig. 8). Die Aufnahmefinger haben solche Abmessungen, dass sie durch die Zugangsschlitze 38 gesteckt werden können und in Eingriff mit den Aktuatoröffnungen 48 kommen. Vorzugsweise sind die Aufnahmefinger 72 konvergierend angeordnet. Das bedeutet, dass sie aufeinander zu weisend ausgerichtet sind, und zwar um einen Winkel β von etwa 10 Grad. Dieser Winkel ist so gewählt, dass die Aufnahmefinger 72 in der Stellung der Revisionszange 7, wenn die Achsstümpfe 42, 43 ihre Montageposition (s. Fig. 3b) erreicht haben, etwa parallel stehen zum einfacheren Abnehmen.

## Patentansprüche

1. Gelenkprothese mit einem Beugescharnier, das von einer Scharniergabel und einem Achsbolzen (4) gebildet ist, der zwei Achsstümpfe (42, 43) umfasst, die zum Einsetzen in einer in einem Koppelstück (3) eingezogenen Montagestellung und nach Implantation durch Bewegung in Achsrichtung in fluchtende Scharnierbohrungen der Scharniergabel in einer expandierten Stellung angeordnet sind,
**dadurch gekennzeichnet, dass**
der Achsbolzen (4) zwei Lagerbereiche (41) an seinen Enden und einen dazwischen liegenden Fügebereich (40) aufweist, und er im Fügebereich (41) längs einer in Achsrichtung verlaufenden Ebene (49) getrennt ist, welche den Mantel des Achsbolzens (4) an zwei Stellen schneidet.

2. Gelenkprothese nach Anspruch 1, **dadurch gekennzeichnet, dass** die Ebene (49) eine Mittelebene des Achsbolzens (4) ist.

3. Gelenkprothese nach Anspruch 1, **dadurch gekennzeichnet, dass** die Ebene (49) aus mehreren Teilebenen besteht.

4. Gelenkprothese nach einem der Ansprüche 1 bis 3, **dadurch gekennzeichnet, dass** die Achsstümpfe (42, 43) formgleich sind.

5. Gelenkprothese nach einem der Ansprüche 1 bis 4, **dadurch gekennzeichnet, dass** der Fügebereich (41) der Achsstümpfe (42, 43) Halbzapfen (44, 45) sind.

6. Gelenkprothese nach einem der Ansprüche 1 bis 5, **dadurch gekennzeichnet, dass** die Achsstümpfe (42, 43) im Querschnitt halbmondförmig ausgebildet sind.

7. Gelenkprothese nach einem der Ansprüche 1 bis 6, **dadurch gekennzeichnet, dass** das Koppelstück (3) in seinem zur Aufnahme des Fügebereichs (41) bestimmten Bereich ein Trennelement (30) mit einer unrunden Durchbrechung für die Achsstümpfe (42, 43) aufweist.

8. Gelenkprothese nach einem der Ansprüche 1 bis 7, **dadurch gekennzeichnet, dass** am Koppelstück (3) eine Aufnahme für eine Verschiebesicherung (63) der Achsstümpfe (42, 43) ausgebildet ist.

9. Gelenkprothese einem der vorangehenden Ansprüche, **dadurch gekennzeichnet, dass** an dem Koppelstück (3) für jeden Achsstumpf (42, 43) ein Zugangsschlitz (38) ausgebildet ist, und ein Spreizinstrument (5) vorgesehen ist, welches zum Eingreifen in die Achsstümpfe (42, 43) durch die Zugangsschlitze (38) ausgebildet ist.

10. Gelenkprothese nach Anspruch 9, **dadurch gekennzeichnet, dass** das Spreizinstrument (5) einen Drahtbügel (50) aufweist, dessen freie Enden (51) zum Einstecken in die Zugangsschlitze (38) ausgebildet sind, wobei die freien Enden (51) vorzugsweise in einem Winkel von 30 Grad bis 70 Grad zueinander stehen und weiter vorzugsweise in seinem von den Enden (51) wegweisenden hinteren Bereich eine Schlaufe (52) in die entgegengesetzte Richtung führt.

11. Gelenkprothese nach Anspruch 9 oder 10, **dadurch gekennzeichnet, dass** eine Schiebehülse (54) vorgesehen ist, die aus einer Ruhestellung, bei der die freien Enden (51) nahe beieinander stehen, in eine Spreizposition verschieblich ist, bei der die freien Enden (51) voneinander wegbewegt sind.

12. Gelenkprothese nach Anspruch 11, **dadurch gekennzeichnet, dass** das Spreizinstrument (5) eine Befestigungsführung (6) aufweist.

13. Gelenkprothese nach Anspruch 12, **dadurch gekennzeichnet, dass** an der Befestigungsführung (6) ein Gewinde (62) angeordnet ist, in das eine Sperrschraube (63) eingedreht ist.

14. Gelenkprothese nach Anspruch 13, **dadurch gekennzeichnet, dass** die Länge der Sperrschraube (63) größer ist als der Abstand zwischen Befestigungsführung (6) und dem Achsbolzen (4).

15. Gelenkprothese nach einem der Ansprüche 11 bis 14, **dadurch gekennzeichnet, dass** die Schiebehülse (54) einen umlaufenden radialen Kragen (56) aufweist, der an seiner zur Befestigungsführung weisenden Seite eine Ausnehmung (55) aufweist.

16. Gelenkprothese nach einem der Ansprüche 9 bis 15, **dadurch gekennzeichnet, dass** eine Revisionszange (7) vorgesehen ist, die an ihrem hinteren Ende Handgriffe (70) und an ihrem vorderen Ende Aufnahmefinger (71) aufweist, die zum Einstecken in die Zugangsschlitze (38) ausgebildet und im Querschnitt oval sind.
